# EUROPEAN PATENT SPECIFICATION

(11) **EP 4 534 121 B1**
(45) Date of publication and mention of the grant of the patent: **20.05.2026**
(21) Application number: 24204823.9
(22) Date of filing: 04.10.2024
(51) Int. Cl.: A61M 5/00, G09B 23/28

(54) **GRIPS FOR INJECTOR TESTING SYSTEMS**
GRIFFE FÜR INJEKTORTESTSYSTEME
POIGNÉES POUR SYSTÈMES DE TEST D'INJECTEUR

(30) Priority: 06.10.2023 US 202363588550 P; 26.09.2024 US 202418898528
(43) Date of publication of application: 09.04.2025
(73) Proprietor: Illinois Tool Works Inc., Glenview IL 60025 (US)
(72) Inventor: DIEP, Sonny, Glenview, 60025 (US)
(74) Representative: HGF

(56) References cited:
- WO-A1-2021/023668
- CN-U- 206 847 862
- US-A1- 2022 134 010
- US-A1- 2023 173 177

## Description

### RELATED APPLICATIONS

The present application claims the benefit of U.S. Provisional Patent Application Serial No. 63/588,550, filed October 6, 2023, entitled "GRIPS FOR INJECTOR TESTING SYSTEMS."

### FIELD OF THE DISCLOSURE

This disclosure relates generally to injection device testing, and more particularly, to grips for injection testing systems.

### BACKGROUND

Injection testing systems may test one or more aspects of injection devices, including autoinjectors, for aspects such as cap removal force, plunger actuation force, injection depth, injection time, needle retraction, and/or delivered dose.

US 2022/0134010 A1 discloses a testing device for testing the moving force of a plunger of a syringe comprising a fixture according to a prior art of the invention.

### SUMMARY

Grips for injection testing systems are disclosed, substantially as illustrated by and described in connection with at least one of the figures, as set forth more completely in the claims.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG. 1 is an example injection testing system to perform testing of injection devices, in accordance with aspects of this disclosure.
FIG. 2 is a block diagram of an example injection testing system including an injection needle positioner and grips, in accordance with aspects of this disclosure.
FIG. 3A and 3B are perspective views of an example grip that may be used to implement one of the grips of FIG. 2.
FIG. 4A is a bottom plan view and FIG. 4B is a top plan view of the example grip of FIGS. 3A and 3B.
FIG. 5 is a side view of the example grip of FIGS. 3A and 3B.
FIG. 6A and 6B are perspective views of an example injection needle positioner having example grips attached to mounting blocks, in accordance with aspects of this disclosure.
FIG. 7 is a bottom view of the example injection needle positioner of FIG. 6 in the closed position when coupled to the mounting brackets in a first orientation.
FIG. 8 is a bottom view of the example injection needle positioner of FIG. 6 in the closed position when coupled to the mounting brackets in a second orientation.

The figures are not necessarily to scale. Wherever appropriate, similar or identical reference numerals are used to refer to similar or identical components.

### DETAILED DESCRIPTION

Injection needle testing devices that measure the dosage delivered by the injection needle, particularly by autoinjectors, include an injection needle positioner that is configured to position an injector (e.g., an autoinjector) securely and in a position suitable for various forms of testing and measurements of the injector. Conventional injection needle testing systems are unable to easily interface with certain types of injectors due to the geometry of the injector.

Disclosed example injection needle testing devices and injection testing systems include one or more grips and that provide excellent gripping characteristics, including supporting a range of injector geometries. Some example injection needle testing systems include an injection needle positioner capable of interfacing with multiple injector geometries, including symmetrical and asymmetrical geometries, with a single pair of grips.

Disclosed example injection needle positioner include: first and second mounting brackets; a first grip, comprising: a first aperture configured to interface with the first mounting bracket; and a first grip face on a lateral side of the first grip, the first grip face comprising: a first section toward a first end of the first grip and having a first profile; and a second section toward a second end of the first grip, relative to the first section, and having a second profile different than the first profile; and a second grip, comprising: a second aperture configured to interface with the second mounting bracket; and a second grip face on a lateral side of the second grip.

In some example injection needle positioners, the second grip face comprises a first section toward a first end of the second grip and having a first profile and a second section toward a second end of the second grip, relative to the first section, and having a second profile different than the first profile. In some injection needle positioners, the second profile of the first grip comprises a recessed gripping groove configured to interface with an injector having an asymmetric geometry. In some injection needle positioners, the second profile of the second grip comprises a recessed gripping groove configured to interface with an injector having an asymmetric geometry.

According to the invention, the first aperture extends from the first end of the first grip to the second end of the first grip, such that the aperture can interface with the first or second mounting bracket from either end of the first grip. In some example injection needle positioners, the second aperture extends from a first end of the second grip to a second end of the second grip, such that the aperture can interface with the first or second mounting bracket from either end of the second grip.

In some example injection needle positioners, the first profile of the first grip is configured to interface with an injector having a symmetric geometry. In some example injection needle positioners, the first profile of the second grip comprises is configured to interface with an injector having a symmetric geometry. In some example injection needle positioners, the symmetrically shaped injector has a cylindrical, elliptical, or semi-square geometry.

In some example injection needle positioners, at least one of the first grip face or the second grip face comprises a slip-resistant coating. In some example injection needle positioners, the slip-resistant coating is surfalloy.

In some example injection needle positioners, each grip lock further comprises one or more accessory mounting holes oriented parallel to the central aperture. In some example injection needle positioners, the accessory mounting holes are configured to interface with one or more customization plates.

In some example injection needle positioners, each grip further comprises a top and bottom mounting hole oriented perpendicular to the central aperture and configured to align with the mounting openings on the mounting bracket. In some example injection needle positioners, each mounting hole is threaded and configured to interface with a mounting screw. In some example injection needle positioners, each grip further comprises a directional indicator configured to point in a predetermined direction when installed in a corresponding configuration.

Disclosed example injection needle positioner include: a positioning plate having a first side configured to contact an injector having a second side opposite the first side; an injection needle positioner configured to position the injector in contact with the first side of the positioning plate, the injection needle positioner comprising: first and second mounting brackets; a first grip, comprising: a first aperture configured to interface with the first mounting bracket; and a first grip face on a lateral side of the first grip, the first grip face comprising: a first section toward a first end of the first grip and having a first profile; and a second section toward a second end of the first grip, relative to the first section, and having a second profile different than the first profile; and a second grip, comprising: a second aperture configured to interface with the second mounting bracket; and a second grip face on a lateral side of the second grip an injector actuator configured to actuate the injector to expel contents of the injector via a needle of the injector.

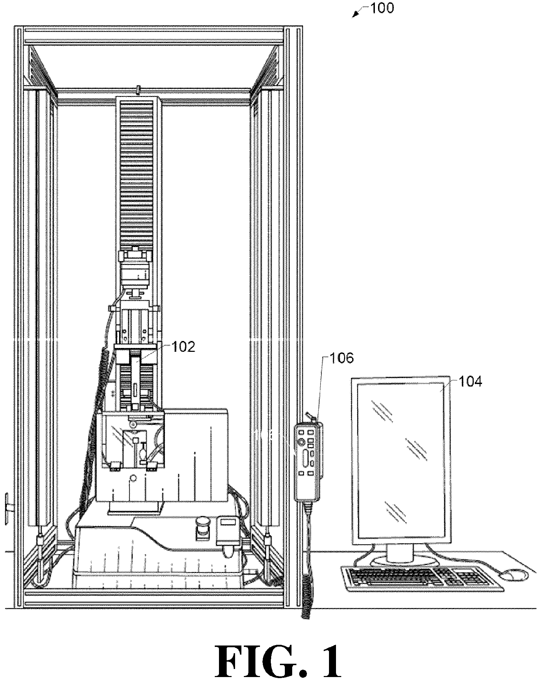

FIG. 1 is an example injection testing system 100 to perform testing on injection devices, such as an autoinjector 102. The example injection testing system 100 may be configured, for example, to perform some or all tests to evaluate requirements of the ISO 11608-5 standard. The example injection testing system 100 may be, for example, a universal testing system configured for injection testing.

The example injection testing system 100 of FIG. 1 includes positioning device(s) (e.g., to position the autoinjector 102 in one or more positions and/or orientations for automated testing), actuator(s) (e.g., to actuate components of the autoinjector 102, to actuate the positioning device(s), to position and/or orient test devices, etc.), and/or sensors to measure aspects of the autoinjector 102 during testing (e.g., load sensors to measure actuation force(s), auditory sensors to detect audible events), mass scales to measure an expelled dose, displacement and/or position sensors to trigger testing steps and/or measure displacement of components of the autoinjector 102, etc.). The example injection testing system 100 further includes one or more user interface devices, such as the displays 104 and input devices 106.

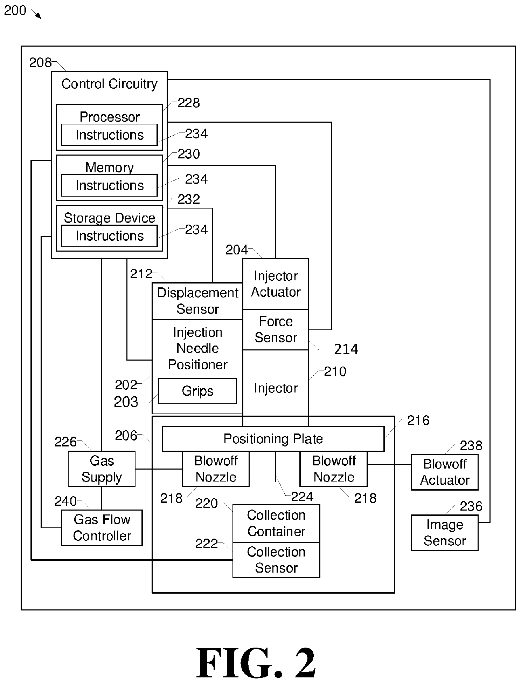

FIG. 2 is a block diagram of an example injection testing system 200 including an injection needle blowoff apparatus. The example injection testing system 200 may be used to implement some or all of the components of the injection testing system 100 of FIG. 1.

The example injection testing system 200 includes an injection needle positioner 202, an injector actuator 204, an injection collector 206, and control circuitry 208. The injection needle positioner 202 positions and/or orients an injector 210 (e.g., an autoinjector) for one or more tests in the injection testing system 200. For example, the injection needle positioner 202 may grasp the injector 210 and move and/or rotate the injector 210 for testing. The injection needle positioner may grasp the injector 210 through two or more grips 203. The injection needle positioner 202 and/or the position of the injector 210 may be measured by one or more displacement sensor(s) 212, which provides displacement and/or position information to the control circuitry 208.

The injector actuator 204 actuates one or more aspects of the injector 210, such as a plunger or other injection mechanism of the injector 210. The force applied by the injector actuator 204 may by measured by a force sensor 214, which provides force measurements to the control circuitry 208.

The injection collector 206 includes a loading surface (e.g., a positioning plate 216), blowoff nozzles 218, and a collection container 220. The collection container 220 and the injector 210 are positioned such that, when the injector 210 is actuated to expel fluid contained in the injector 210, the fluid is expelled into the collection container 220. A collection sensor 222 measures the mass and/or volume collected in the collection container 220, and provides a mass or volume measurement to the control circuitry 208.

The positioning plate 216 allows a needle 224 of the injector 210 to extend through the positioning plate 216 toward the collection container 220. The positioning plate 216 may block a body of the injector 210 from extending through the positioning plate 216 using appropriately sized apertures for the needle 224 and the body of the injector 210. To test the dispensing of the contained fluid, the injection needle positioner 202 may position the injector 210 to contact or abut the positioning plate 216, such that the needle 224 extends through the aperture of the positioning plate 216. When the injector 210 is positioned, the injector 210 may be actuated (e.g., manually, or automatically via the injector actuator 204) to expel the contents of the injector 210 into the collection container 220.

While the examples disclosed herein use the positioning plate 216 as a loading surface, other examples may use different types of loading surfaces against which the injector 210 can be actuated to expose the needle 224 and/or expel the contents of the injector 210. For example, rods or other structural members which are positioned to contact a body of the injector 210 on a top side of the loading surface, and to avoid obstructing the needle 224 may be used. In some such examples, the blowoff nozzles 218 may be coupled to another surface, or otherwise adjustably supported, within the injection collector 206 adjacent the bottom side of the loading surface and/or adjacent the location the needle 224.

At the completion of actuation of the injector 210, the blowoff nozzles 218 are controlled to blow the last drop of fluid from at or near the tip of the needle into the collection container 220. A gas supply 226 provides a gas, such as nitrogen or air, to the blowoff nozzles 218. The gas supply 226 may be, for example, a compressed gas source, a pneumatic pump, or a blower. The blowoff nozzles 218 may be positioned and/or oriented to adjust a location at which the blowoff gas strikes the needle 224, and/or may be positioned and/or oriented to cause the blowoff gas to strike the needle 224 over a range of needle lengths.

The example control circuitry 208 may be a general-purpose computer, a laptop computer, a tablet computer, and/or any other type of processing system configured to communicate with the sensors and actuators of the injection testing system 200. For example, the control circuitry 208 includes a processor 228, memory 230, and a storage device 232. The example processor 228 may be any general purpose central processing unit (CPU) from any manufacturer. In some other examples, the processor 228 may include one or more specialized processing units, such as RISC processors with an ARM core, graphic processing units, digital signal processors, and/or system-on-chips (SoC). The processor 228 executes machine readable instructions 234 that may be stored locally at the processor (e.g., in an included cache or SoC), in the memory (e.g., a random access memory or other volatile memory, a read only memory or other non-volatile memory such as FLASH memory, and/or in the storage device 232. The example storage device 232 may be a hard drive, a solid state storage drive, a hybrid drive, a RAID array, and/or any other mass data storage device.

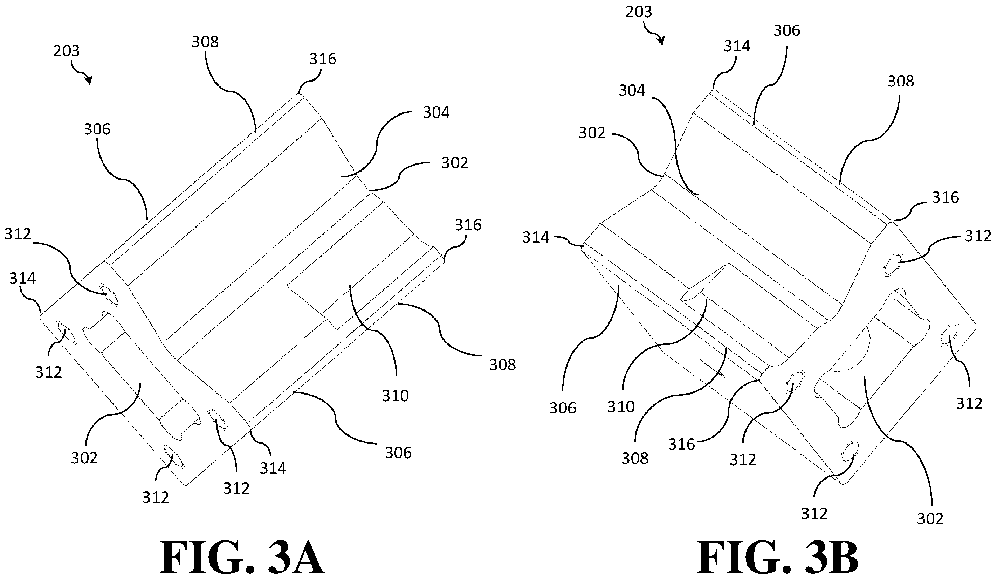

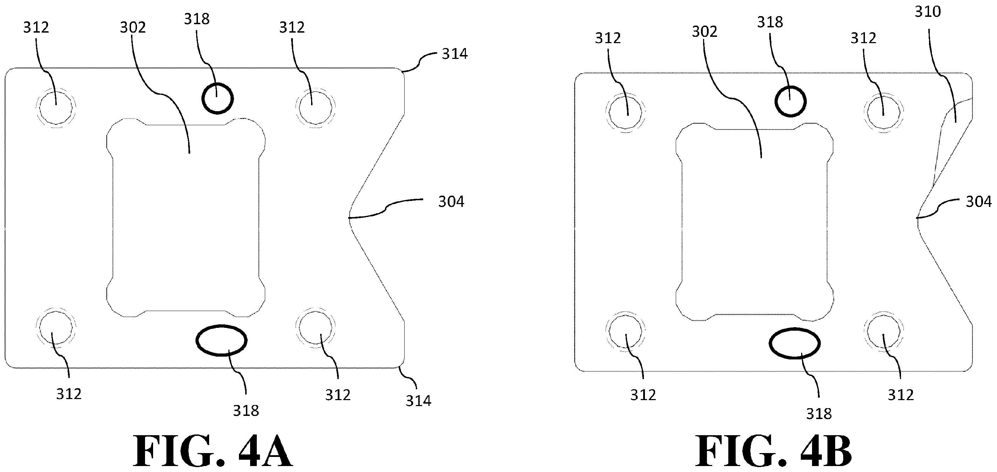

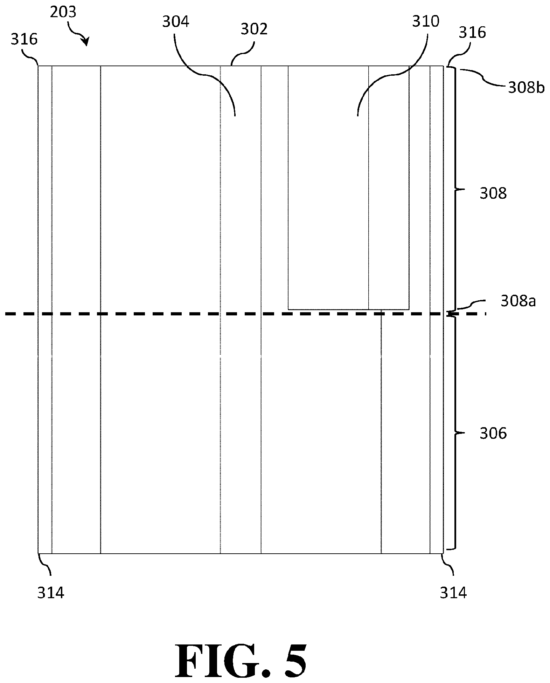

FIG. 3A and 3B are perspective views of an example grip 203. As discussed above, injection needle positioner 202 may include one or more grips 203 through which injection needle positioner 202 may grasp an injector 210. FIG. 4A is a bottom plan view of the example grip 203 of FIGS. 3A and 3B, and FIG. 4B is a top plan view of the example grip 203 of FIGS. 3A and 3B. FIG. 5A is a side view of the example grip of FIGS. 3A and 3B.

The grip 203 may include one or more apertures 302 through which the grip 203 can interface with or be coupled to the injection needle positioner 202. For example, the grip 203 may include single aperture 302. The aperture 302 may be configured to interface with or be coupled to a mounting bracket 606 of the injection needle positioner 202. The aperture 302 may extend throughout a portion of the length of the grip 203 or throughout the entire length of the grip 203. For example, the aperture 302 may extend from a first end 314 of the grip 203 to a second end 316 of the grip 203, such that the aperture 302 can interface with a mounting bracket 606 from either end of the grip 203. The size and shape of the aperture 302 may be varied. For example, the aperture 302 may be square, semi-square, rectangular, circular, elliptical, or any other suitable shape.

The example grip 203 further includes a grip face 304 on a lateral side of the grip 203. The example grip face 304 includes two or more sections, such as a first section 306 and a second section 308. The first section 306 extends from or toward a first end 314 of the grip 203, and the second section 308 extends from or toward a second end 316 of the grip 203. Each section 306, 308 has a profile that is configured to interface with an injector 210. The first section 306 and the second section 308 of the example grip 203 have different profiles to grip injectors having different profiles. However, the grip 203 may be replaced with a grip having a consistent profile (e.g., a flat face) over all or a portion of the length of the grip face.

As described above, the profile of each section of the example grip 203 is configured to interface with an injector 210. To that end, the shape of the profile may be varied depending on the shape of the injector 210. For example, the example grip section 308 is configured to interface with an injector 210 that has an asymmetric geometry (e.g., a specific asymmetric geometry that is incompatible with gripping by the first section 306). To that end, the example second section 308 includes a recessed gripping groove 310 that is configured to interface with an injector 210 that has an asymmetric geometry. The example recessed gripping groove 310 extends from the second end 316 of the grip 203. In some examples, the recessed gripping groove 310 extends across at least a portion of the length of the second section 308. In other examples, the recessed gripping groove extends across the entire length of the second section 308 (e.g., extending from first end 308a to a second end 308b of the second section).

**In** other examples, the grip section 306 is configured to interface with an injector 210 that has a symmetric geometry. Example symmetric geometries of the injector 210 include cylindrical, elliptical, and semi-square geometries. For example, the profile of the first section 306 of example grip 203 is defined by a v-shaped, symmetrical surface. In other examples, the first section 306 is defined by other suitable symmetrical surface geometries, such as a semicircular geometry. While an example angle of the surface is illustrated in FIGS. 3A, 3B, and 4, different angles may be used.

**In** some examples, the grip 203 is be made from a material that is uncoated. In other examples, at least a portion of grip 203 includes a slip-resistant coating. The slip-resistant coating of example grip 203 is configured to enhance the stability of injector 210 when engaged by two grips 203. Various slip-resistant coatings may be included. In some examples the slip-resistant coating is a surfalloy coating, rubber, brake lining materials, and/or any other appropriate coating material. Additionally or alternatively, the grip face 304 may be knurled, or otherwise textured to increase friction between the grip 203 and the injector 210 without coating. The grip 202, including the grip face 304, may also be bead blasted, passivated, and/or otherwise treated to improve aesthetics and/or corrosion resistance.

The grip 203 may include one or more accessory mounting holes 312. The one or more accessory mounting holes 312 may be located on either end 314, 316 of the grip 203 and may be oriented parallel to the aperture 302. In some examples, the accessory mounting holes 312 extends from a first end 314 to a second end 316 of example grip 203. In other examples, the accessory mounting holes 312 extend from a first end 314 or from a second end 316, but only extend a portion of the length of the example grip 203. The accessory mounting holes 312 may be threaded and configured to interface with a threaded mounting screw.

The accessory mounting holes 312 may be configured to interface with or couple to various accessories to either side of example grip 203. For example, the accessory mounting holes 312 may be configured to interface with one or more customization plates. In some examples, the one or more customization plates are configured to alter, customize, and/or replace the profile of the first and/or second sections 306 and 308 of the grip face 304. For example, a custom plate may be attached to each of the grips 203 via the accessory mounting holes 312. The profile of the custom plate may be configured to engage the injector 210 prior to engagement of the injector 210 by the grips 203, which causes the custom plate to perform the gripping of the injector 210. The custom plates may be coated for improved grip as disclosed above. As described above, the shape of the profile may be configured to interface with an injector 210 having a certain geometry, such as a symmetrical or asymmetrical geometry.

The grips 203 may include more or fewer accessory mounting holes 312 than shown in the illustrated examples. For instance, the grips 203 may include one or more additional alignment holes 318, which may be engaged by the custom plates and/or by an alignment object (e.g., a pin) which aids in appropriate alignment of the custom plates. The alignment holes 318 may have one or more predetermined shapes and/or positions on the grips 203 to promote alignment and/or prevent incorrect orientation of the custom plates or other objects.

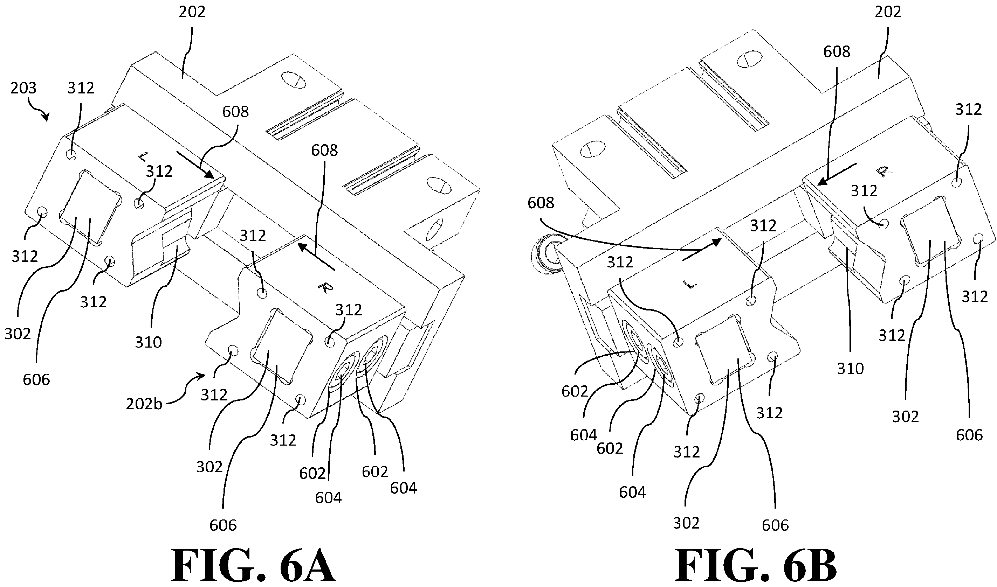

As illustrated in FIGS. 6A and 6B, the example grips 203 further include directional indicators 608. In the illustrated example, the directional indicators 608 point toward a direction in which the grips 203 close, to thereby guide an operator in properly orienting the grips 203 during installation. If the operator orients the grips 203 incorrectly, then the directional indicators 608 may point in the wrong direction.

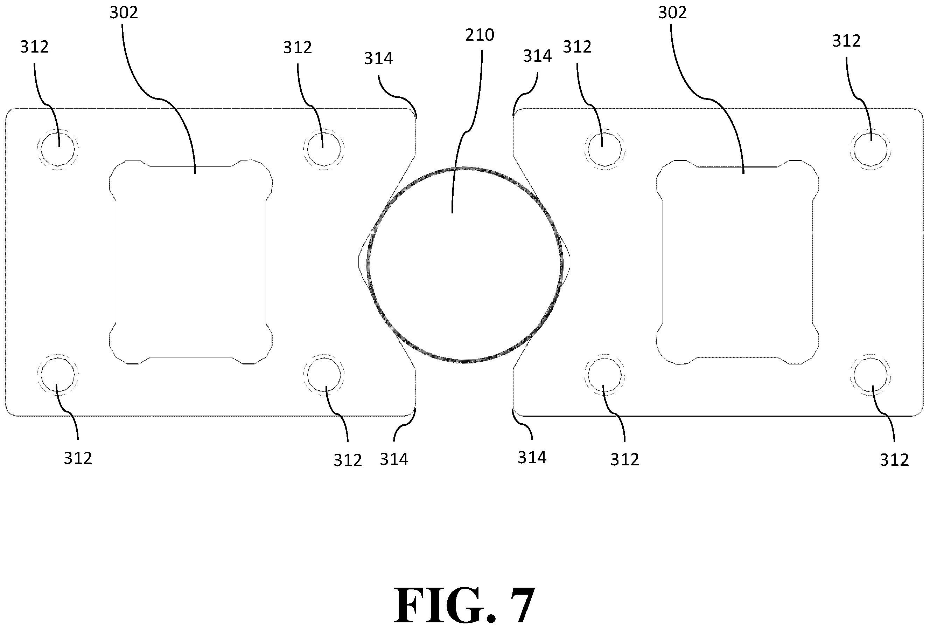

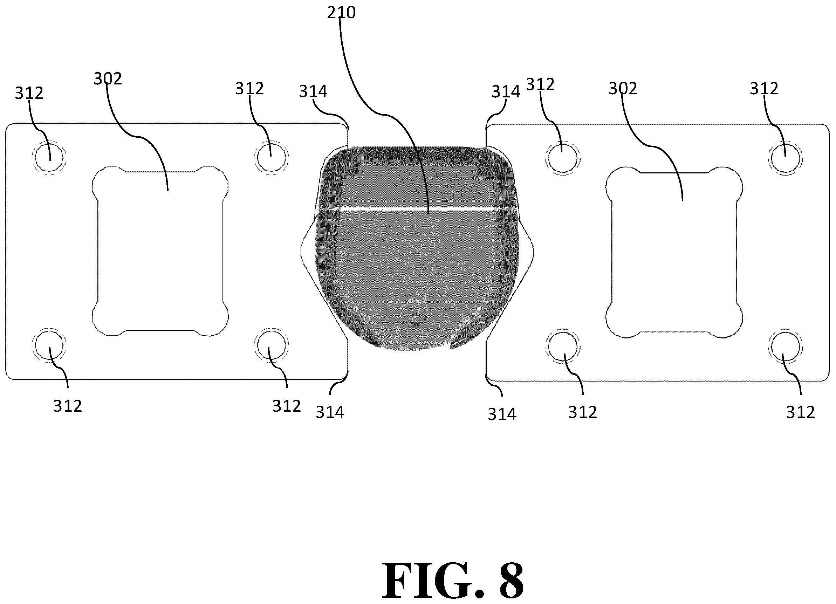

FIG. 6A and 6B are perspective views of an example injection needle positioner 202, including grips 203 coupled to the injection needle positioner 202 through mounting brackets 606. FIG. 7 is a bottom view of the example injection needle positioner of FIG. 6 where the grips 203 are in the closed position when coupled to the mounting brackets in a first orientation. FIG. 8 is a bottom view of the example injection needle positioner of FIG. 6 where the grips 203 are in the closed position when coupled to the mounting brackets in a second orientation. As shown in FIG. 6A and 6B, the example grip 203 includes one or more mounting holes 602 configured to interface with or couple to a mounting bracket 606. The mounting holes may be oriented perpendicular to the aperture 302. In some examples, the mounting holes 602 are threaded and configured to interface with a threaded mounting screw 604 to couple to mounting bracket 606. In further examples, one or more threaded mounting screws 604 may interface with one or more mounting openings located on the mounting bracket 606 to securely couple the grip 203 to the mounting bracket 606.

As shown in FIG. 6A and 6B, the example injection needle positioner 202 includes two grips 203 coupled to the injection needle positioner 202 through two mounting brackets 606. FIG. 6A and 6B illustrate an example injection needle positioner 202 including two grips 203 oriented in an open position. When in the open position as illustrated in FIG. 6A and 6B, there is space between the grips 203 such that the grips 203 are oriented to receive an injector 210 (e.g., an autoinjector). The two grips 203 may be moved laterally towards one into a closed position as illustrated in FIG. 7. When in the closed position as illustrated in FIG. 7, the example grips 203 are configured to securely grip an injector 210. As illustrated in FIG. 6A, 6B, and 7, each grip 203 of the injection needle positioner 202 may include some or all of the components of the grip 203 as described above and illustrated in FIG. 1-5.

The present methods and systems may be realized in hardware, software, and/or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein. As used herein, the term "non-transitory machine-readable medium" is defined to include all types of machine readable storage media and to exclude propagating signals.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made without departing from the scope of the invention as defined by the claims. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed.

## Claims

1. An injection needle positioner (202), comprising:
first and second mounting brackets (606);
a first grip (203), comprising:
a first aperture (302) configured to interface with the first mounting bracket; and
a first grip face on a lateral side of the first grip, the first grip face comprising:
a first section (306) toward a first end of the first grip and having a first profile; and
a second section (308) toward a second end of the first grip, relative to the first section, and having a second profile different than the first profile;
a second grip (203), comprising:
a second aperture (302) configured to interface with the second mounting bracket; and
a second grip face on a lateral side of the second grip; and being **characterised in that** the first aperture (302) extends from the first end of the first grip to the second end of the first grip, such that the aperture can interface with the first or second mounting bracket from either end of the first grip, and/or
wherein the second aperture (302) extends from a first end of the second grip to a second end of the second grip, such that the aperture can interface with the first or second mounting bracket from either end of the second grip.

2. The injection needle positioner of claim 1, wherein the second grip face comprises a first section toward a first end of the second grip and having a first profile and a second section toward a second end of the second grip, relative to the first section, and having a second profile different than the first profile.

3. The injection needle positioner of claim 1, wherein the second profile of the first grip comprises a recessed gripping groove (310) configured to interface with an injector having an asymmetric geometry.

4. The injection needle positioner of claim 2, wherein the second profile of the second grip comprises a recessed gripping groove (310) configured to interface with an injector having an asymmetric geometry.

5. The injection needle positioner of claim 1, wherein the first profile of the first grip is configured to interface with an injector (210) having a symmetric geometry.

6. The injection needle positioner of claim 1, wherein the first profile of the second grip comprises is configured to interface with an injector (210) having a symmetric geometry.

7. The injection needle positioner of claim 5, wherein the symmetrically shaped injector has a cylindrical, elliptical, or semi-square geometry.

8. The injection needle positioner of claim 6, wherein the symmetrically shaped injector has a cylindrical, elliptical, or semi-square geometry.

9. The injection needle positioner of claim 1, wherein at least one of the first grip face or the second grip face comprises a slip-resistant coating.

10. The injection needle positioner of claim 9, wherein the slip-resistant coating is surfalloy.

11. The injection needle positioner of claim 1, wherein each grip lock further comprises one or more accessory mounting holes oriented parallel to the central aperture, and optionally
wherein the accessory mounting holes are configured to interface with one or more customization plates.

12. The injection needle positioner of claim 1, wherein each grip further comprises a top and bottom mounting hole oriented perpendicular to the central aperture and configured to align with the mounting openings on the mounting bracket, and optionally
wherein each mounting hole is threaded and configured to interface with a mounting screw.

13. The injection needle positioner of claim 1, wherein each grip further comprises a directional indicator configured to point in a predetermined direction when installed in a corresponding configuration.

14. An injection needle testing system (100, 200), comprising:
a positioning plate (216) having a first side configured to contact an injector having a second side opposite the first side;
an injection needle positioner (202) configured to position the injector in contact with the first side of the positioning plate, the injection needle positioner comprising:
first and second mounting brackets (606);
a first grip (203), comprising:
a first aperture (302) configured to interface with the first mounting bracket; and
a first grip face on a lateral side of the first grip, the first grip face comprising:
a first section (306) toward a first end of the first grip and having a first profile; and
a second section (308) toward a second end of the first grip, relative to the first section, and having a second profile different than the first profile;
a second grip, comprising:
a second aperture (302) configured to interface with the second mounting bracket; and
a second grip face on a lateral side of the second grip;
wherein the first aperture (302) extends from the first end of the first grip to the second end of the first grip, such that the aperture can interface with the first or second mounting bracket from either end of the first grip, and/or
wherein the second aperture (302) extends from a first end of the second grip to a second end of the second grip, such that the aperture can interface with the first or second mounting bracket from either end of the second grip; and
an injector actuator (204) configured to actuate the injector to expel contents of the injector via a needle of the injector.

## Patentansprüche

1. Positioniervorrichtung für Injektionsnadeln (202), die Folgendes aufweist:
erste und zweite Befestigungsklammern (606);
einen ersten Griff (203), der Folgendes aufweist:
eine erste Öffnung (302), die so ausgebildet ist, dass sie mit der ersten Befestigungsklammer schnittstellenmäßig verbunden ist; und
eine erste Grifffläche an einer lateralen Seite des ersten Griffs, wobei die erste Grifffläche Folgendes aufweist:
einen ersten Abschnitt (306) in Richtung eines ersten Endes des ersten Griffs und mit einem ersten Profil; und
einen zweiten Abschnitt (308) in Richtung eines zweiten Endes des ersten Griffs in Bezug auf den ersten Abschnitt und mit einem zweiten Profil, das sich von dem ersten Profil unterscheidet;
einen zweiten Griff (203), der Folgendes aufweist:
eine zweite Öffnung (302), die so ausgebildet ist, dass sie mit der zweiten Befestigungsklammer schnittstellenmäßig verbunden ist; und
eine zweite Grifffläche an einer lateralen Seite des zweiten Griffs; und **dadurch gekennzeichnet, dass**
die erste Öffnung (302) sich von dem ersten Ende des ersten Griffs zu dem zweiten Ende des ersten Griffs erstreckt, sodass die Öffnung mit der ersten oder zweiten Befestigungsklammer von jedem Ende des ersten Griffs schnittstellenmäßig verbunden werden kann, und/oder
wobei sich die zweite Öffnung (302) von einem ersten Ende des zweiten Griffs zu einem zweiten Ende des zweiten Griffs erstreckt, sodass die Öffnung mit der ersten oder zweiten Befestigungsklammer von jedem Ende des zweiten Griffs schnittstellenmäßig verbunden werden kann.

2. Positioniervorrichtung für Injektionsnadeln nach Anspruch 1, wobei die zweite Grifffläche einen ersten Abschnitt in Richtung eines ersten Endes des zweiten Griffs und mit einem ersten Profil sowie einen zweiten Abschnitt in Richtung eines zweiten Endes des zweiten Griffs relativ zu dem ersten Abschnitt und mit einem zweiten Profil, das sich von dem ersten Profil unterscheidet, aufweist.

3. Positioniervorrichtung für Injektionsnadeln nach Anspruch 1, wobei das zweite Profil des ersten Griffs eine vertiefte Griffnut (310) aufweist, die so ausgebildet ist, dass sie mit einem Injektor mit einer asymmetrischen Geometrie schnittstellenmäßig verbunden ist.

4. Positioniervorrichtung für Injektionsnadeln nach Anspruch 2, wobei das zweite Profil des zweiten Griffs eine vertiefte Griffnut (310) aufweist, die so ausgebildet ist, dass sie mit einem Injektor mit einer asymmetrischen Geometrie schnittstellenmäßig verbunden ist.

5. Positioniervorrichtung für Injektionsnadeln nach Anspruch 1, wobei das erste Profil des ersten Griffs so ausgebildet ist, dass es mit einem Injektor (210) mit einer symmetrischen Geometrie schnittstellenmäßig verbunden ist.

6. Positioniervorrichtung für Injektionsnadeln nach Anspruch 1, wobei das erste Profil des zweiten Griffs aufweist so ausgebildet ist, dass es mit einem Injektor (210) mit einer symmetrischen Geometrie schnittstellenmäßig verbunden ist.

7. Positioniervorrichtung für Injektionsnadeln nach Anspruch 5, wobei der symmetrisch geformte Injektor eine zylindrische, elliptische oder halbquadratische Geometrie aufweist.

8. Positioniervorrichtung für Injektionsnadeln nach Anspruch 6, wobei der symmetrisch geformte Injektor eine zylindrische, elliptische oder halbquadratische Geometrie aufweist.

9. Positioniervorrichtung für Injektionsnadeln nach Anspruch 1, wobei mindestens eine der ersten Grifffläche oder der zweiten Grifffläche eine rutschfeste Beschichtung aufweist.

10. Positioniervorrichtung für Injektionsnadeln nach Anspruch 9, wobei die rutschfeste Beschichtung Surfalloy ist.

11. Positioniervorrichtung für Injektionsnadeln nach Anspruch 1, wobei jede Griffverriegelung ferner ein oder mehrere Zubehörbefestigungslöcher aufweist, die parallel zu der zentralen Öffnung ausgerichtet sind, und optional
wobei die Zubehörbefestigungslöcher so ausgebildet sind, dass sie mit einer oder mehreren Anpassungsplatten schnittstellenmäßig verbunden sind.

12. Positioniervorrichtung für Injektionsnadeln nach Anspruch 1, wobei jeder Griff ferner ein oberes und ein unteres Montageloch aufweist, die jeweils senkrecht zu der zentralen Öffnung ausgerichtet und so ausgebildet sind, dass sie sich an den Befestigungsöffnungen an der Befestigungsklammer ausrichten, und optional
wobei jedes Befestigungsloch mit einem Gewinde versehen und so konfiguriert ist, dass es mit einer Befestigungsschraube schnittstellenmäßig verbunden ist.

13. Positioniervorrichtung für Injektionsnadeln nach Anspruch 1, wobei jeder Griff ferner einen Richtungsanzeiger aufweist, der so ausgebildet ist, dass er in eine vorbestimmte Richtung zeigt, wenn er in einer entsprechenden Konfiguration installiert ist.

14. Injektionsnadel-Prüfsystem (100, 200), das Folgendes aufweist:
eine Positionierungsplatte (216), die eine erste Seite aufweist, die so ausgebildet ist, dass sie mit einem Injektor in Kontakt kommt, wobei der Injektor eine der ersten Seite gegenüberliegende zweite Seite aufweist;
eine Positioniervorrichtung für Injektionsnadeln (202), die so ausgebildet ist, dass sie den Injektor in Kontakt mit der ersten Seite der Positionierungsplatte positioniert, wobei die Positioniervorrichtung für Injektionsnadeln Folgendes aufweist:
erste und zweite Befestigungsklammern (606);
einen ersten Griff (203), der Folgendes aufweist:
eine erste Öffnung (302), die so ausgebildet ist, dass sie mit der ersten Befestigungsklammer schnittstellenmäßig verbunden ist;
und
eine erste Grifffläche an einer lateralen Seite des ersten Griffs, wobei die erste Grifffläche Folgendes aufweist:
einen ersten Abschnitt (306) in Richtung eines ersten Endes des ersten Griffs und mit einem ersten Profil; und
einen zweiten Abschnitt (308) in Richtung eines zweiten Endes des ersten Griffs in Bezug auf den ersten Abschnitt und mit einem zweiten Profil, das sich von dem ersten Profil unterscheidet;
einen zweiten Griff, der Folgendes aufweist:
eine zweite Öffnung (302), die so ausgebildet ist, dass sie mit der zweiten Befestigungsklammer schnittstellenmäßig verbunden ist; und
eine zweite Grifffläche an einer lateralen Seite des zweiten Griffs;
wobei sich die erste Öffnung (302) von dem ersten Ende des ersten Griffs bis zu dem zweiten Ende des ersten Griffs erstreckt, sodass die Öffnung mit der ersten oder zweiten Befestigungsklammer von jedem Ende des ersten Griffs schnittstellenmäßig verbunden werden kann, und/oder
wobei sich die zweite Öffnung (302) von einem ersten Ende des zweiten Griffs zu einem zweiten Ende des zweiten Griffs erstreckt, sodass die Öffnung mit der ersten oder zweiten Befestigungsklammer von jedem Ende des zweiten Griffs schnittstellenmäßig verbunden werden kann; und
einen Injektoraktuator (204), der so ausgebildet ist, dass er den Injektor betätigt, um den Inhalt des Injektors über eine Nadel des Injektors auszutreiben.

## Revendications

1. Dispositif de positionnement d'aiguille d'injection (202), comprenant :
des premiers et deuxièmes supports de montage (606) ;
une première poignée (203), comprenant :
une première ouverture (302) configurée pour servir d'interface avec le premier support de montage ; et
une première face de prise sur un côté latéral de la première poignée, la première face de prise comprenant :
une première section (306) vers une première extrémité de la première poignée et ayant un premier profil ; et
une deuxième section (308) vers une deuxième extrémité de la première poignée, par rapport à la première section, et ayant un deuxième profil différent du premier profil ;
une deuxième poignée (203), comprenant :
une deuxième ouverture (302) configurée pour servir d'interface avec le deuxième support de montage ; et
une deuxième face de prise sur un côté latéral de la deuxième poignée ; et étant **caractérisée en ce que**
la première ouverture (302) s'étend de la première extrémité de la première poignée à la deuxième extrémité de la première poignée, de sorte que l'ouverture peut servir d'interface avec le premier ou le deuxième support de montage à partir de l'une ou l'autre extrémité de la première poignée, et/ou
dans lequel la deuxième ouverture (302) s'étend depuis une première extrémité de la deuxième poignée jusqu'à une deuxième extrémité de la deuxième poignée, de telle sorte que l'ouverture peut servir d'interface avec le premier ou le deuxième support de montage depuis l'une ou l'autre extrémité de la deuxième poignée.

2. Dispositif de positionnement d'aiguille d'injection selon la revendication 1, dans lequel la deuxième face de prise comprend une première section vers une première extrémité de la deuxième poignée et ayant un premier profil et une deuxième section vers une deuxième extrémité de la deuxième poignée, par rapport à la première section, et ayant un deuxième profil différent du premier profil.

3. Dispositif de positionnement d'aiguille d'injection selon la revendication 1, dans lequel le deuxième profil de la première poignée comprend une rainure de prise évidée (310) configurée pour servir d'interface avec un injecteur ayant une géométrie asymétrique.

4. Dispositif de positionnement d'aiguille d'injection selon la revendication 2, dans lequel le deuxième profil de la deuxième poignée comprend une rainure de prise évidée (310) configurée pour servir d'interface avec un injecteur ayant une géométrie asymétrique.

5. Dispositif de positionnement d'aiguille d'injection selon la revendication 1, dans lequel le premier profil de la première poignée est configuré pour servir d'interface avec un injecteur (210) ayant une géométrie symétrique.

6. Dispositif de positionnement d'aiguille d'injection selon la revendication 1, dans lequel le premier profil de la deuxième poignée est configuré pour servir d'interface avec un injecteur (210) ayant une géométrie symétrique.

7. Dispositif de positionnement d'aiguille d'injection selon la revendication 5, dans lequel l'injecteur de forme symétrique a une géométrie cylindrique, elliptique ou semi-carrée.

8. Dispositif de positionnement d'aiguille d'injection selon la revendication 6, dans lequel l'injecteur de forme symétrique a une géométrie cylindrique, elliptique ou semi-carrée.

9. Dispositif de positionnement d'aiguille d'injection selon la revendication 1, dans lequel l'au moins une de la première face de prise ou de la deuxième face de prise comprend un revêtement antidérapant.

10. Dispositif de positionnement d'aiguille d'injection selon la revendication 9, dans lequel le revêtement antidérapant est du surfalloy.

11. Dispositif de positionnement d'aiguille d'injection selon la revendication 1, dans lequel chaque verrou de préhension comprend en outre un ou plusieurs trous de montage d'accessoire orientés parallèlement à l'ouverture centrale, et facultativement
dans lequel les trous de montage d'accessoires sont configurés pour s'interfacer avec une ou plusieurs plaques de personnalisation.

12. Dispositif de positionnement d'aiguille d'injection selon la revendication 1, dans lequel chaque poignée comprend en outre un trou de montage supérieur et inférieur orienté perpendiculairement à l'ouverture centrale et configuré pour s'aligner avec les ouvertures de montage sur le support de montage, et facultativement
dans lequel chaque trou de montage est fileté et configuré pour s'interfacer avec une vis de montage.

13. Dispositif de positionnement d'aiguille d'injection selon la revendication 1, dans lequel chaque poignée comprend en outre un indicateur directionnel configuré pour pointer dans une direction prédéterminée lorsqu'il est installé dans une configuration correspondante.

14. Système de test d'aiguille d'injection (100, 200), comprenant :
une plaque de positionnement (216) ayant un premier côté configuré pour être en contact avec un injecteur ayant un deuxième côté opposé au premier côté ;
un dispositif de positionnement d'aiguille d'injection (202) configuré pour positionner l'injecteur en contact avec le premier côté de la plaque de positionnement, le dispositif de positionnement d'aiguille d'injection comprenant :
des premiers et deuxièmes supports de montage (606) ;
une première poignée (203), comprenant :
une première ouverture (302) configurée pour servir d'interface avec le premier support de montage ; et
une première face de prise sur un côté latéral de la première poignée, la première face de prise comprenant :
une première section (306) vers une première extrémité de la première poignée et ayant un premier profil ; et
une deuxième section (308) vers une deuxième extrémité de la première poignée, par rapport à la première section, et ayant un deuxième profil différent du premier profil ;
une deuxième poignée, comprenant :
une deuxième ouverture (302) configurée pour s'interfacer avec le deuxième support de montage ; et
une deuxième face de préhension sur un côté latéral de la deuxième poignée ;
dans lequel la première ouverture (302) s'étend de la première extrémité de la première poignée à la deuxième extrémité de la première poignée, de sorte que l'ouverture peut servir d'interface avec le premier ou le deuxième support de montage à partir de l'une ou l'autre extrémité de la première poignée, et/ou
dans lequel la deuxième ouverture (302) s'étend depuis une première extrémité de la deuxième poignée jusqu'à une deuxième extrémité de la deuxième poignée, de telle sorte que l'ouverture peut servir d'interface avec le premier ou le deuxième support de montage depuis l'une ou l'autre extrémité de la deuxième poignée ; et
un actionneur d'injecteur (204) configuré pour actionner l'injecteur afin d'expulser le contenu de l'injecteur via une aiguille de l'injecteur.
